# EUROPÄISCHE PATENTSCHRIFT

(11) **EP 1 525 900 B1**
(45) Veröffentlichungstag und Bekanntmachung des Hinweises auf die Patenterteilung: **03.05.2006**
(21) Anmeldenummer: 03024343.0
(22) Anmeldetag: 24.10.2003
(51) Int. Cl.: A61N 1/32, C12M 1/42, H01C 1/14

(54) **Verfahren zur Herstellung eines elektrisch kontaktierbaren Bereichs auf einem dotierten Polymer und nach dem Verfahren herstellbarer Formkörper**
Process for the formation of electrically contactable conductors on a conductive polymer and products obtainable therewith
Procédé de réalisation d'un élément conducteur sur un polymère conducteur et éléments obtenus à partir dudit procédé

(43) Veröffentlichungstag der Anmeldung: 27.04.2005
(73) Patentinhaber: Amaxa GmbH, 50829 Köln (DE)
(72) Erfinder: Müller-Hertmann, Herbert, 50823 Köln (DE); Siebenkotten, Gregor, 50226 Frechen-Königsdorf (DE); Fernbach, Ewald, 50129 Bergheim-Büsdorf (DE)

(56) Entgegenhaltungen:
- EP-A- 1 100 295
- WO-A-00/57680
- DE-A- 10 208 188
- US-A- 4 765 874

## Beschreibung

Die Erfindung betrifft ein Verfahren zur Herstellung zumindest eines elektrisch kontaktierbaren Bereichs auf einem mit einem leitfähigen Material dotierten Polymer, bei dem auf das Polymer ein Kontaktmaterial unter Druck aufgebracht wird, welches bei 23°C einen geringeren spezifischen Widerstand als das Polymer aufweist. Die Erfindung betrifft ferner einen Formkörper aus mit leitfähigem Material dotiertem Polymer, mit zumindest einem kontaktierbaren Bereich, innerhalb dessen auf das Polymer ein Kontaktmaterial aufgebracht ist, welches bei 23°C einen geringeren spezifischen Widerstand als das Polymer aufweist.

Elektrisch leitfähige Polymere sind bekannt und erfreuen sich zunehmender Beliebtheit, insbesondere auch als Material zur Herstellung von Elektroden zur Erzeugung elektrischer Felder für unterschiedlichste Anwendungen.

Aus der EP 0 307 007 B1 ist beispielsweise eine Anordnung für elektrische Einrichtungen bekannt, die leitfähige Elemente mit unterschiedlichen spezifischen Widerständen aufweisen. Ein Widerstandselement aus einem leitfähigen Polymer, d. h. einem Gemisch aus einem organischen Polymer und einem leitfähigen Füllstoff, das bei 23°C einen relativ hohen spezifischen Widerstand von 1 - 500.000 Ohm·cm aufweist, wird dabei mit einer Kontaktschicht versehen, die aus einem leitfähigen Material besteht, dessen spezifischer Widerstand geringer als der des Widerstandselements ist, d.h. zwischen 2,5 × 10⁻⁵ und 1 × 10⁻³ Ohm·cm liegt. Die Kontaktschicht besteht ebenfalls aus einem leitfähigen Polymer, das mit einem Metall, beispielsweise Silber, oder einem kohlenstoffhaltigen Material, beispielsweise Graphit, dotiert ist. Die Kontaktschicht ist in Form von bandförmigen Elektroden, die fingerartig ineinander greifen, auf das Widerstandselement aufgebracht. Als Anschlusselemente sind Stromschienen vorgesehen, die aus einem gestreckten Metallnetz bestehen und um die Kontaktschicht bzw. die hieraus gebildeten Elektroden herumgefaltet sind. Dabei bilden die Randbereiche der Elektroden die Kontaktfläche. Zwar wird bei dieser Lösung der Eingangswiderstand der Widerstandsschicht durch das Aufbringen einer Kontaktschicht mit geringerem spezifischen Widerstand herabgesetzt, jedoch besteht diese Kontaktschicht ihrerseits aus einem dotierten Polymer und weist daher ebenfalls noch einen relativ hohen Eingangswiderstand auf. Dies liegt vor allem dann vor, wenn bei einem Spritzen der Kontaktschicht eine Entmischung an deren Oberfläche auftritt. Die Kontaktierung der Kontaktschicht erfolgt hier ferner über anliegende Stromschienen, d.h. gestreckte Metallnetze, und ist somit nicht mit einer punktförmigen Kontaktierung vergleichbar. Es gibt aber viele Anwendungen, bei denen aufgrund der spezifischen Erfordernisse oder der räumlichen Gegebenheiten eine punktförmige Kontaktierung, beispielsweise über Federkontakte, erforderlich ist. Eine punktförmige Kontaktierung der hier offenbarten Kontaktschicht würde beim Anlegen sehr hoher Spannungen aber zu einem Einbrennen der Anschlusselemente an den Kontaktsstellen führen.

Gerade im Bereich biologischer Anwendungen sind Elektroden aus leitfähigen Kunststoffen im Vergleich zu herkömmlichen Metallelektroden vorteilhaft, da aus den Metallelektroden bei einer elektrischen Entladung Metallionen freigesetzt werden können. Bei der Behandlung von lebenden Zellen, beispielsweise bei der Elektroporation oder der Elektrofusion, können die in die entsprechende Zellsuspension abgegebenen Metallionen entweder in geringer Konzentration zu einer unerwünschten Stimulierung der Zellen oder in höherer Konzentration zu einer toxischen Wirkung führen. So konnte beispielsweise bei der Verwendung von Aluminiumelektroden ein negativer Effekt auf die behandelten Zellen nachgewiesen werden, der durch freigesetzte Al³⁺-lonen verursacht wird (Loomis-Husselbee et al., Biochem J 1991, 277 (Pt 3), 883 - 885). Darüber hinaus kann es bei der Verwendung von Metallelektroden zur Bildung von Metallhydroxyden oder Komplexen von Metallionen mit biologischen Makromolekülen kommen (Stapulionis, Bioelectrochem Bioenerg 1999, 48 (1), 249 - 254), was ebenfalls häufig unerwünscht ist.

Aus der DE 102 08 188 A1 sind Behälter mit Elektroden aus dotierten Polymeren bekannt. Bei den dotierten Polymeren handelt es sich um Polymere, denen leitfähige Stoffe wie beispielsweise Kohlefasern, Graphit, Russ oder Kohlenstoff-Nanotubes beigemischt sind. Solche dotierten Polymere weisen zwar im Vergleich zu intrinsisch leitenden Polymeren eine geringere Leitfähigkeit auf, haben aber den Vorteil, dass sie spritzfähig sind, d. h. im Spritzgussverfahren verarbeitet werden können. Solche dotierten Polymere sind also vielseitig anwendbar und ermöglichen dabei die kostengünstige Herstellung von Elektroden im Spritzgussverfahren. Ein Problem bei solchen Elektroden besteht aber darin, dass beim Spritzvorgang eine Entmischung stattfindet, so dass die Konzentration der leitfähigen Dotierung an der Oberfläche der Elektroden relativ gering ist. Entsprechende Elektroden haben daher einen sehr hohen Eingangswiderstand, was zur Folge hat, dass sehr hohe Spannungen angelegt werden müssen, um einen ausreichenden Stromfluss zu erreichen. Bei der üblichen punktförmigen Kontaktierung dieser Elektroden, beispielsweise durch Federkontakte, kommt es aber aufgrund der hohen angelegten Spannungen ebenfalls zu einem Einbrennen der Kontakte in die Elektrodenoberfläche, so dass diese unbrauchbar werden.

Aus der US 4 765 874 ist eine Elektrode für Elektrolysen bekannt, die einen Grundkörper aus Kunststoff aufweist, der mit einem leitfähigen Material dotiert ist. Zur Erhöhung der mechanischen Stabilität kann bei dieser Polymerelektrode ein Streckmetall oder ein Metallgitter unter Druck und Wärmezufuhr in die Elektrodenoberfläche eingedrückt werden.

Aus der DE 101 16 211 A1 ist ferner eine Vorrichtung zur Fusion von lebenden Zellen im elektrischen Feld bekannt, bei der die Elektroden ebenfalls aus einem dotierten Kunststoff, d. h. einem Kunststoff, der mit Kohlenstoff gefüllt ist, hergestellt sind. Die Elektroden sind punktuell über Kontaktpunkte und entsprechende Leitungen mit einer Spannungsquelle verbunden. Auch hier besteht also der Nachteil, dass ein Einbrennen in die Oberfläche der Kunststoffelektroden eintritt, wenn höhere Spannungen angelegt werden sollten als die, die für eine Elektrodenfusion erforderlich sind. Um beispielsweise für besondere Anwendungen der Elektroporation ausreichende Feldstärken zu erreichen, müssten an die Elektroden wesentlich höhere Spannungen angelegt werden. So können beispielsweise für das Einbringen von biologisch aktiven Molekülen in den Zellkem lebender Zellen Feldstärken von 2-10 kV/cm erforderlich sein. Die zur Erreichung solcher Feldstärken erforderliche Spannung würde bei der punktförmigen Kontaktierung der bekannten Polymerelektroden auch hier zu einem Einbrennen der Kontaktstellen führen.

Es ist daher Aufgabe der Erfindung, die genannten Nachteile zu vermeiden und ein Verfahren der eingangs genannten Art bereitzustellen, welches eine effektive Reduzierung des Eingangswiderstands des Polymers in dem kontaktierbaren Bereich auf einfache und kostengünstige Weise ermöglicht, sowie einen Formkörper der eingangs genannten Art zu schaffen, der einen geringen Eingangswiderstand aufweist und einfach und kostengünstig herzustellen ist.

Erfindungsgemäß wird die Aufgabe durch ein Verfahren der eingangs genannten Art gelöst, bei dem als Kontaktmaterial eine Folie unter Einwirkung von erhöhter Temperatur, die über der Erweichungstemperatur des dotierten Polymers liegt, auf das Polymer aufgebracht wird. Durch das dichte Aufbringen des Kontaktmaterials, das einen geringeren spezifischen Widerstand als das Polymer aufweist, wird der Eingangswiderstand des dotierten Polymers effektiv herabgesetzt, wobei es durch den engen Kontakt des Kontaktmaterials mit dem Polymer praktisch zu einer "Verschmelzung" der beiden Materialien kommt und dadurch ein intensiver Kontakt zu der leitenden Dotierung des Polymers hergestellt wird. Die Dotierung des Polymers, die beispielsweise aus Kohlefasern und/oder Graphit bestehen kann, liegt in dem Polymer in einer bestimmten Konzentration vor, beispielsweise 75 Gew.-%, die gewährleistet, dass das dotierte Polymer eine ausreichende Leitfähigkeit aufweist. Da es beispielsweise beim Spritzen des dotierten Polymers in eine Spritzform zu einer Entmischung an der Oberfläche des Polymers kommt, d. h. die Dotierung an der Oberfläche des Materials in deutlich geringerer Konzentration vorliegt als darunter, muss das Kontaktmaterial derart dicht auf das Polymer aufgebracht werden, dass es mit der leitfähigen Dotierung zumindest annähernd in der bestimmten Konzentration in Kontakt tritt. Auf diese Weise wird die entmischte Zone an der Oberfläche, in der nur wenig leitfähiges Material vorliegt, überbrückt, so dass der Eingangswiderstand effektiv verringert werden kann. Um den Eingangswiderstand, insbesondere für Anwendungen, bei denen sehr hohe Feldstärken zwischen 2 und 10 kV/cm zwischen zwei Polymerelektroden erreicht werden müssen, effektiv zu verringern, muss das Kontaktmaterial ferner so dicht auf das Polymer aufgebracht werden, dass das Kontaktmaterial eine so ausreichende Verbindung zu der Dotierung des Polymers herstellt, dass die Temperatur an der Kontaktfläche zwischen Polymer und Kontaktmaterial während des Durchleitens von elektrischem Strom unterhalb des Erweichungspunktes des dotierten Polymers bleibt. Durch diese Maßnahmen kann ein mit leitfähigem Material dotiertes Polymer in dem kontaktierbaren Bereich auch punktuell, d.h. beispielsweise mit einem Draht oder einem Federkontakt, effektiv kontaktiert werden, ohne dass sich die Kontakte bei hohen angelegten Spannungen in die Oberfläche des Polymers einbrennen.

Im Idealfall können die beiden Materialen miteinander verschmelzen. Darüber hinaus erfolgt ein Eindrücken oder Eindringen des Kontaktmaterials in die Oberfläche des Polymers , so dass der Kontakt zur Dotierung des Polymers, d.h. dem leitfähigen Material, so intensiv ist, dass der Eingangswiderstand reduziert werden kann. Durch die Verwendung einer Folie wind das erfindungsgemäße Verfahren vereinfacht , wobei gleichzeitig eine sehr dünne und dicht anliegende Kontaktschicht erzeugt wird.

Da das Kontaktmaterial unter Druck aufgebracht wird, ist der Kontakt zwischen dem Kontaktmaterial und dem dotierten Polymer noch intensiver, was zu einer weiteren vorteilhaften Verringerung des Eingangswiderstands des Polymers führt. Dabei kann das Kontaktmaterial vorzugsweise auf das Polymer aufgepresst werden.

In besonders vorteilhafter Ausgestaltung der Erfindung ist ferner vorgesehen, dass die Oberfläche des Polymers vor dem Aufbringen des Kontaktmaterials zumindest teilweise durch mechanische und/oder chemische Behandlung vergrößert wird. Dabei kann die Oberfläche beispielsweise durch mechanische Verletzung aufgeraut werden. Diese Maßnahmen erleichtern das Aufbringen des Kontaktmaterials und sorgen darüber hinaus für eine noch engere Verzahnung der beiden Materialien.

Das Kontaktmaterial sollte vorzugsweise bei 23°C einen sehr geringen spezifischen Widerstand aufweisen, der beispielsweise unterhalb von 1 x 10⁻⁵ Ohm cm liegt. Vorzugsweise sollte der spezifische Widerstand 1 x 10⁻⁶ bis 2 x 10⁻⁶ Ohm-cm betragen.

In weiterer vorteilhafter Ausgestaltung der Erfindung ist vorgesehen, dass das Kontaktmaterial eine Metallfolie, insbesondere eine Kupferfolie, oder eine Folie aus intrinsisch leitendem Kunststoff ist. Kupfer ist als Kontaktmaterial besonders vorteilhaft, da es bei 23°C einen sehr geringen spezifischen Widerstand von ungefähr 1,7 x 10⁻⁶ Ohm·cm aufweist, leicht zu verarbeiten und kostengünstig ist.

Der intrinsisch leitende Kunststoff kann in der zuvor beschriebenen Ausführungsform beispielsweise ein Polyanilin, Polyacetylen, Poly-para-phenylen, Poly-para-phenylensulfid, Polypyrrol oder Polythiophen sein oder eines oder mehrere dieser Polymere als Hauptbestandteil enthalten.

In einer besonderen Ausführungsform des erfindungsgemäßen Verfahrens kann das Kontaktmaterial mittels eines Prägestempels auf das Polymer aufgeprägt, d.h. beispielsweise durch Heißprägen aufgebracht werden. Dabei können das Kontaktmaterial und zumindest ein Teil des Polymers auf eine Temperatur erwärmt werden, die oberhalb der Erweichungstemperatur des Polymers liegt. Das Kontaktmaterial wird dann mittels des Prägestempels unter Druck auf die Oberfläche des Polymers gepresst. Nach dem Abkühlen auf eine Temperatur, die unterhalb der Erweichungstemperatur liegt, wird der Prägestempel schließlich wieder abgehoben. Das Kontaktmaterial und/oder zumindest ein Teil des Polymers und/oder der Prägestempel können beim Heißprägen auf eine Temperatur zwischen 100 und 300 °C erwärmt werden. Der Prägestempel kann mit einem Druck zwischen 50 und 100 N/mm² oder zwischen 100 und 500 N/mm² beaufschlagt werden. Alternativ kann der Prägestempel während der Prägephase auch zunächst mit einem Druck von 50 bis 100 N/mm², vorzugsweise 70 bis 90 N/mm², und dann, während der Abkühlphase, mit einem Druck von 100 bis 500 N/mm², vorzugsweise 350 bis 450 N/mm², beaufschlagt werden. Das Aufprägen des Kontaktmaterials unter Wärmezufuhr, d.h. beispielsweise das Heißprägen, kann dabei zumindest teilweise im Vakuum oder in einer Stickstoffatmosphäre durchgeführt werden. In jedem Fall handelt es sich beim Heißprägen um eine effektive und kostengünstige Ausführungsform des erfindungsgemäßen Verfahrens.

In vorteilhafter Ausgestaltung der Erfindung ist vorgesehen, dass das Kontaktmaterial über eine haftvermittelnde Schicht, die vorzugsweise einen geringen spezifischen Widerstand aufweist, auf das Polymer aufgebracht wird, um den Kontakt zwischen dem Kontaktmaterial und dem Polymer zu verbessern. Die haftvermittelnde Schicht kann dabei in vorteilhafter Ausgestaltung ein leitfähiges Material sein, beispielsweise ein elektrisch leitender Klebstoff oder Ähnliches.

Erfindungsgemäß wird die Aufgabe ferner durch einen Formkörper der eingangs genannten Art gelöst, bei dem das Kontaktmaterial eine auf das Polymer heißgeprägte Folie ist. Ein solcher Formkörper weist einen deutlich verringerten Eingangswiderstand auf und kann problemlos auch punktuell, d.h. beispielsweise mit einem Draht oder einem Federkontakt, effektiv kontaktiert werden, ohne dass sich die Kontakte bei hohen angelegten Spannungen in die Oberfläche des Polymers einbrennen. Dabei muss der Kontakt zwischen dem Kontaktmaterial und der Dotierung des Polymers so intensiv sein, dass die Temperatur an der Kontaktfläche zwischen Kontaktmaterial und Polymer während des Durchleitens von elektrischem Strom unterhalb des Erweichungspunktes des dotierten Polymers liegt. Bei dem erfindungsgemäßen Formkörper ist dies dadurch gewährleistet, dass das Kontaktmaterial eine enge, d.h. sehr intensive, Verbindung zu der Dotierung des Polymers darstellt, die den Eingangswiderstand des Polymers effektiv verringert.

Das Kontaktmaterial kann in vorteilhafter Ausgestaltung der Erfindung ein Metall, vorzugsweise Kupfer, oder ein intrinsisch leitender Kunststoff sein, da diese Materialien einen geringen spezifischen Widerstand aufweisen und leicht zu verarbeiten sind. Das Kontaktmaterial sollte einen spezifischen Widerstand bei 23°C unterhalb von 1 x 10⁻⁵ Ohm·cm, vorzugsweise von 1 x 10⁻⁶ bis 2 x 10⁻⁶ Ohm·cm, aufweisen.

Um den Eingangswiderstand des erfindungsgemäßen Formkörpers weiter zu verringern, kann zwischen dem Polymer und dem Kontaktmaterial eine kohlenstoffhaltige Schicht, vorzugsweise eine Graphitschicht, oder eine haftvermittelnde Schicht, die vorzugsweise einen geringen spezifischen Widerstand aufweist, angeordnet sein, welche jeweils einen intensiven Kontakt zu der leitfähigen Dotierung des Polymers vermitteln. Die haftvermittelnde Schicht kann dabei in vorteilhafter Ausgestaltung ein leitfähiges Material sein, beispielsweise ein elektrisch leitender Klebstoff oder Ähnliches.

Das Polymer ist vorzugsweise mit Kohlefasern, Graphit, Ruß und/oder Kohlenstoff-Nanotubes dotiert, wobei die Dotierung vorteilhafterweise in einer Konzentration von 50 bis 80 Gew.-% vorliegt. Das dotierte Polymer sollte dabei einen spezifischen Widerstand bei 23 °C von ungefähr 0,4 bis 1,0 Ohm·cm, vorzugsweise 0,46 Ohm·cm, aufweisen. Wird ein solches dotiertes Polymer mittels Spritzgießen verarbeitet, so verringert sich die Konzentration der Dotierung an der Oberfläche durch Entmischung. Der Oberflächenwiderstand ist daher relativ hoch, d. h. er liegt beispielsweise bei 2 bis 10 Ohm, insbesondere 8 bis 8,5 Ohm. Dieser Eingangswiderstand muss durch das dichte Aufbringen des Kontaktmaterials verringert werden, damit der Gesamtwiderstand des erfindungsgemäßen Formkörpers nicht zu hoch ist.

Das Polymer kann beispielsweise Polycarbonat, Polyetheretherketon, Polypropylen, Polyamid vorzugsweise Polyamid 6 oder Polyamid 66, Polyphenylensulfid oder ein Gemisch dieser Polymere sein oder eines oder mehrere dieser Polymere als Hauptbestandteil enthalten.

Der intrinsisch leitende Kunststoff kann beispielsweise ein Polyanilin, Polyacetylen, Poly-para-phenylen, Poly-para-phenylensulfid, Polypyrrol oder Polythiophen sein oder eines oder mehrere dieser Polymere als Hauptbestandteil enthalten.

Der erfindungsgemäße Formkörper ist vorzugsweise als Elektrode oder ähnliches Bauteil ausgebildet, das zum Weiterleiten von elektrischem Strom benötigt wird. Der Formkörper kann auch, vorzugsweise in Form einer Elektrode, Bestandteil einer Küvette oder zumindest eines Reaktionsraums einer Multiwell-Platte sein, beispielsweise für die Elektroporation oder Elektrofusion von lebenden Zellen, insbesondere auch für HT-Anwendungen (HT = high throughput).

Tabelle 1 zeigt einen Vergleich der nach dem erfindungsgemäßen Verfahren hergestellten erfindungsgemäßen Formkörper mit entsprechenden Formkörpern ohne dicht aufgebrachtes Kontaktmaterial. Verglichen wurde das Verhalten dotierter Polymerelektroden bei einer Kontaktierung mit Federkontakten aus Messing. Zunächst wurden Elektroden ohne jegliches Kontaktmaterial oder Zwischenschicht getestet. Bei einer Spannung von 1000 V brannten sich hierbei die Federkontakte deutlich in die Kontaktfläche der Elektroden ein. In einem weiteren Versuch wurden die Elektroden dann in eine Vorrichtung eingeschoben, in der der Kontakt nicht unmittelbar durch die Federkontakte erfolgte, sondern über eine Kupferfolie, die unter leichtem Druck auf die Kontaktfläche der Elektroden aufgebracht war. Auch hier konnte ein Einbrennen der Kupferfolie in die Kontaktfläche des dotierten Polymers beobachtet werden, wenn auch in schwächerer Ausprägung. Bei den Verwendung von aufgepressten Kupferplatten wurde kein Einbrennen beobachtet. Bei den verschiedenen Ausführungsformen der erfindungsgemäßen Formkörper bzw. nach dem erfindungsgemäßen Verfahren hergestellten Elektroden konnte unter gleichen Bedingungen ebenfalls kein ein Einbrennen der Federkontakte oder des Kontaktmaterials in die Kontaktfläche des dotierten Polymers beobachtet werden. Durch das dichte Aufbringen des jeweiligen Kontaktmaterials auf das dotierte Polymer kann also ein Einbrennen eines verwendeten Anschlusselements auch bei einer hohen angelegten Spannung von 1000 V sicher vermieden werden.

**Tabelle 1: Verhindern des Einbrennens in die Oberfläche von Formkörpern in Form von Elektroden aus dotiertem Polymer (Polyamid 6 mit Kohlefasern und Graphit)**

| **Kontaktierung** | **Angelegte Gleichspannung; geflossene Ladung** | **Einbrennen; Oberflächenbeschädigung** |
|---|---|---|
| Federkontakte (Messing) | 1000 V; 5 mC | Ja, deutlich |
| Kupferfolienadapter (ca. 20 mN/mm²) | 1000 V; 5 mC | Ja, schwach |
| Kupferplatten (ca. 2 N/mm²) * | 1000 V; 5 mC | Nein |
| Kupferplatten + Graphit (ca. 2 N/mm²) ** | 1000 V; 5 mC | Nein |
| 55 µm Kupferfolie, heiß geprägt, 100 mm² | 1000 V; 5 mC | Nein |
| 55 µm Kupferfolie, heiß geprägt, 8 mm² | 1000 V; 5 mC | Nein |

| | | |
|---|---|---|
| * Auflagefläche ca. 100 mm² | | |
| ** Graphit: Dichte 1 g/cm³, 1 mm Stärke, 100 mm² Auflagefläche auf Polymer | | |

Die Erfindung wird im weiteren anhand der Figuren beispielhaft näher erläutert.

Es zeigt:
- Figur 1: einen Querschnitt durch eine Polymerküvette, die mit nach dem erfindungsgemäßen Verfahren hergestellten Elektroden ausgestattet ist,
- Figur 2: Balkendiagramme der Stromstärke und des Widerstandes, die einen Vergleich der Leitfähigkeit zwischen Elektroden mit und ohne aufgebrachtes Kontaktmaterial zeigen, wobei die verwendeten Polymerküvetten mit Elektroden aus einem dotierten Polymer (Polyamid 6 mit Kohlefasern und Graphit) ausgestattet sind und das Kontaktmaterial eine mittels Heißprägen aufgebrachte Kupferfolie ist, Stärke der Kupferfolie: 55 µm, Spaltbreite der Küvetten: 1,5 mm, Volumen der in den Küvetten befindlichen Elektrolytlösung: 100 µl, angelegte Spannung: 1000 V Gleichspannung,
A: Elektrolytlösung mit spezifischer Leitfähigkeit von 17,02 mS/cm, n=6
B: Elektrolytlösung mit spezifischer Leitfähigkeit von 17,02 mS/cm, n=2,
- Figur 3: Balkendiagramme der Stromstärke und des Widerstandes eines Vergleichs der Leitfähigkeit von Polymerküvetten mit auf unterschiedliche Kontaktflächen des dotierten Polymers nach dem erfindungsgemäßen Verfahren aufgebrachter Kupferfolie, breit = 100 mm² und schmal = 18 mm², Spaltbreite der Küvetten = 1,5 mm, Volumen der Elektrolytlösung = 100 µl, spezifische Leitfähigkeit der Elektrolytlösung = 12,75 mS/cm, Stärke der Kupferfolie = 55 µm, graue Balken = 1000 V Gleichspannung, schwarze Balken = 500 V Gleichspannung, n = 2,
- Figur 4: eine perspektivische Ansicht einer Küvette mit erfindungsgemäßen Formkörpern und
- Figur 5: eine perspektivische Ansicht der Küvette gemäß Figur 4 mit einer weiteren Ausführungsform erfindungsgemäßer Formkörper.

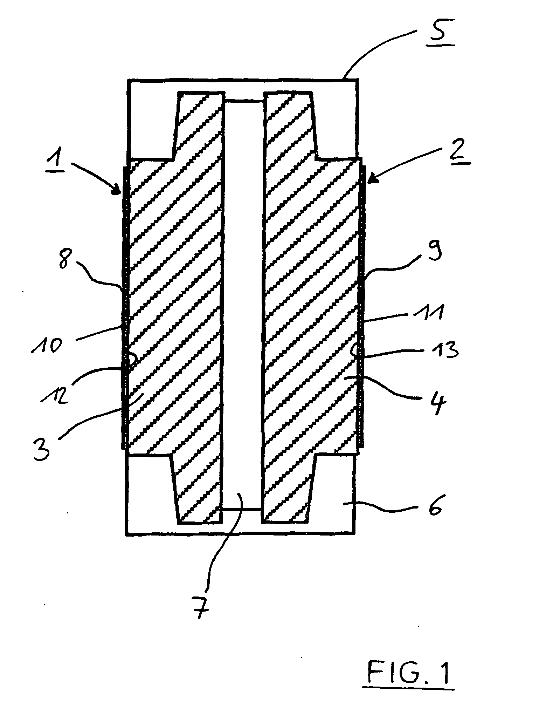

Figur 1 zeigt einen Querschnitt durch zwei erfindungsgemäße Formkörper 1, 2, die in diesem Ausführungsbeispiel als Elektroden 3, 4 einer Küvette 5 ausgebildet sind. Die Küvette 5 besteht aus einem Rahmen 6, der aus einem spritzfähigen Polymer hergestellt ist und der in seinem Bodenbereich zwei gegenüberliegend angeordnete Elektroden 3, 4 aufweist. Die Elektroden 3, 4 bestehen ebenfalls aus einem spritzfähigen Polymer, das mit leitfähigen Stoffen, beispielsweise Kohlefasern und/oder Graphit, dotiert ist. Die parallel gegenüberliegend angeordneten Elektroden 3, 4 schließen einen spaltförmigen Innenraum 7 ein, welcher der Aufnahme einer Flüssigkeit, beispielsweise einer Elektrolytlösung, dient. Beim Anlegen einer elektrischen Spannung an die beiden Elektroden 3, 4 fließt ein elektrischer Strom durch die Flüssigkeit bzw. Elektrolytlösung im Innenraum 7. Beispielsweise können in der Elektrolytlösung lebende Zellen suspendiert sein, in die in der Elektrolytlösung gelöste biologisch aktive Moleküle, beispielsweise Nukleinsäuren oder Proteine, mit Hilfe des durch den Innenraum 7 fließenden elektrischen Stroms eingebracht werden können. Zum Anlegen einer Spannung müssen die Elektroden 3, 4 an ihren Außenseiten 8, 9 mittels eines geeigneten Anschlusselementes, beispielsweise eines Federkontakts, kontaktiert werden. Um ein Einbrennen des Anschlusselements, insbesondere beim Anlegen sehr hoher Spannungen, zu vermeiden und den Eingangswiderstand des Polymers zu reduzieren, ist auf die beiden Außenseiten 8, 9 der Elektroden 3, 4 jeweils ein Kontaktmaterial 10, 11 mit hoher Leitfähigkeit dicht aufgebracht. Das Kontaktmaterial 10, 11 bedeckt dabei im vorliegenden Ausführungsbeispiel jeweils die gesamte jeweils zur Verfügung stehende Kontaktfläche 12, 13 der Elektroden 3, 4. Bei dem Kontaktmaterial 10, 11 kann es sich beispielsweise um eine Kupferfolie oder eine Folie aus einem intrinsisch leitenden Kunststoff handeln. Das Kontaktmaterial 10, 11 sollte jedenfalls einen spezifischen Widerstand bei 23°C von weniger als 1 × 10⁻⁵ Ohm cm aufweisen. Das Kontaktmaterial 10, 11 muss ferner in jedem Fall dicht auf die Kontaktflächen 12, 13 aufgebracht werden, um den hohen Eingangswiderstand der Elektroden 3, 4 effektiv zu reduzieren. Zu diesem Zweck kann das Kontaktmaterial 10, 11, vorzugsweise unter Wärmezufuhr, wobei die Temperatur oberhalb der Erweichungstemperatur des Polymers liegen sollte, und/oder beispielsweise unter Druck aufgepresst oder mittels Heißprägen aufgeprägt werden. Alternativ kann das Kontaktmaterial 10, 11 aber auch mittels einer haftvermittelnden Schicht auf die Kontaktflächen 12, 13 aufgebracht, beispielsweise aufgeklebt, werden. Durch das sehr dichte Aufbringen des Kontaktmaterials 10, 11 auf die Kontaktflächen 12, 13 der Elektroden 3, 4 kommt dieses mit der Dotierung der Elektroden 3, 4, d. h. dem leitfähigen Material innerhalb des Polymers, in engen, d.h. besonders intensiven, Kontakt, so dass das Kontaktmaterial 10, 11 praktisch eine Verbindung zwischen dem hier nicht dargestellten Anschlusselement, beispielsweise einem Federkontakt, und dem leitfähigen Material innerhalb der Elektroden 3, 4 herstellt. Auf diese Weise wird der Eingangswiderstand der Elektroden 3, 4 deutlich verringert, so dass bei hohen Spannungen weniger Energie in Form von Wärme an den Kontaktflächen 12, 13, d.h. dem kontaktierbaren Bereich, frei wird. Die Temperatur an den Kontaktflächen 12, 13 bleibt durch diese Maßnahme unterhalb des Schmelz- oder Erweichungspunktes des Polymers, aus dem die Elektroden 3, 4 hergestellt sind, so dass ein Einbrennen des Anschlusselementes und des Kontaktmaterials verhindert wird. Die nach dem erfindungsgemäßen Verfahren hergestellten erfindungsgemäßen Formkörper 1, 2 können also vorteilhafterweise auch für Anwendungen verwendet werden, bei denen mit hohen Spannungen gearbeitet wird und eine punktförmige Kontaktierung verwendet werden soll bzw. muss.

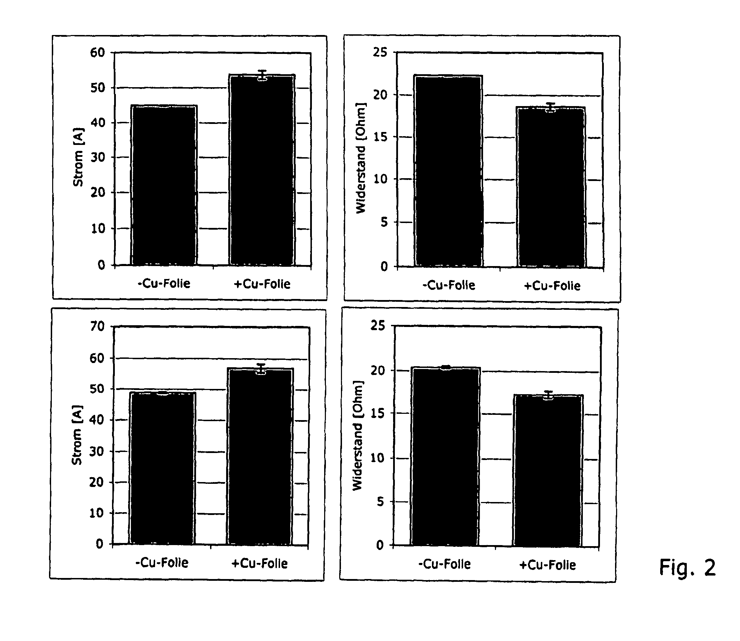

Figur 2 zeigt Vergleiche der Leitfähigkeiten von Polymerküvetten gemäß Figur 1, die mit Elektroden mit mittels Heißprägen aufgebrachten Kupferfolien ausgestattet sind, im Vergleich zu entsprechenden Küvetten mit Elektroden aus dotiertem Polymer ohne Kontaktmaterial bzw. aufgeprägte Kupferfolie. Aus den gezeigten Balkendiagrammen ist anhand des gesamten Stromflusses und des gemessenen Gesamtwiderstandes ersichtlich, dass das auf die Elektroden aufgebrachte Kontaktmaterial die Leitfähigkeit der Elektroden erhöht. In allen Fällen konnte mit Kontaktmaterial (Cu-Folie) ein erhöhter Stromfluss und ein verringerter Gesamtwiderstand gemessen werden. Da der Unterschied zwischen den verglichenen Küvetten ausschließlich in dem auf die Kontaktflächen der Elektroden aufgebrachten Kontaktmaterial besteht, zeigen diese Versuche, dass durch das Kontaktmaterial der Eingangswiderstand der Elektroden verringert werden konnte.

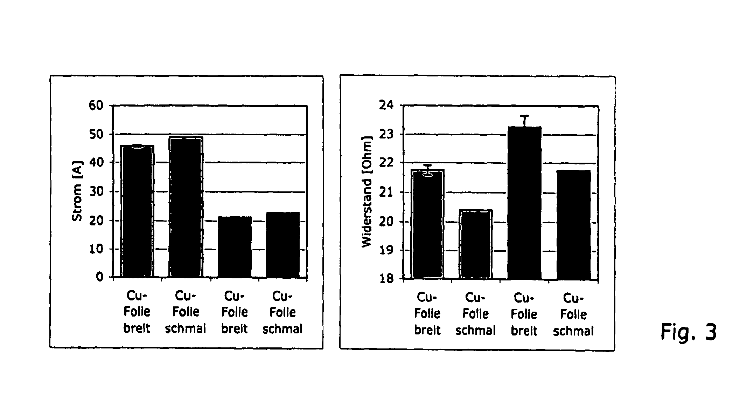

Figur 3 zeigt einen Vergleich zwischen Polymerküvetten gemäß Figur 1, bei denen das Kontaktmaterial, hier eine Kupferfolie, auf unterschiedlich große Kontaktflächen des Polymers dicht aufgebracht ist. Der Vergleich wurde bei unterschiedlichen Spannungen durchgeführt. Überraschenderweise hat sich bei diesen Versuchen herausgestellt, dass eine Verringerung der Kontaktfläche mit einer deutlicheren Reduzierung des Widerstands einhergeht. Die Erklärung für dieses Phänomen liegt möglicherweise darin, dass sich der Anpressdruck bei den schmalen Kupferfolien auf eine geringere Fläche verteilt und somit ein besserer Kontakt zu dem leitfähigen Material innerhalb des Polymers hergestellt wird. Dieser Effekt überwiegt dann möglicherweise den vermuteten negativen Effekt einer geringeren Fläche.

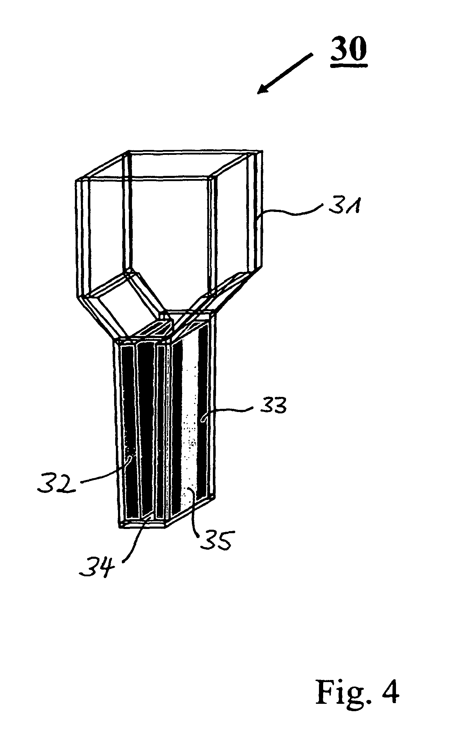

Figur 4 zeigt eine perspektivische Ansicht einer Küvette 30, die aus einem Grundkörper 31 und zwei erfindungsgemäßen Formkörpern 32, 33 besteht. Die Formkörper 32, 33 sind im unteren, verengten Bereich des Grundkörpers 31 angeordnet. Die parallel angeordneten Formkörper 32, 33 schließen dabei einen Spalt 34 ein, der der Aufnahme beispielsweise einer Zellsuspension dient. Wie beim Formkörper 33 ersichtlich, ist auf die Formkörper 32, 33 ein streifenförmiges Kontaktmaterial 35 erfindungsgemäß aufgebracht. Dabei kann es sich beispielsweise um eine aufgeprägte Kupferfolie oder Ähnliches handeln. Die Formkörper 32, 33 können beispielsweise aus einem mit Kohlefasern und Graphit dotiertem Polymer, beispielsweise Polyamid 6 oder Polyamid 66, bestehen. Beim Anlegen einer elektrischen Spannung an das Kontaktmaterial 35 wirken die Formkörper 32, 33 als Elektroden, so dass in dem Spalt 34 ein elektrisches Feld entsteht. Mit Hilfe dieses elektrischen Feldes können beispielsweise biologisch aktive Moleküle mittels Elektroporation in die Zellen eingebracht oder mittels Elektrofusion fusioniert werden.

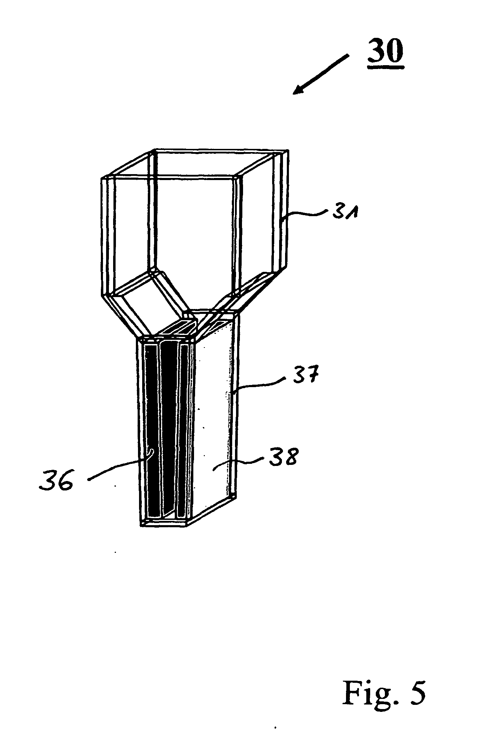

Figur 5 zeigt eine perspektivische Ansicht der Küvette 30 gemäß Figur 4, die ebenfalls im unteren Bereich zwei Elektroden 36, 37 aufweist. Im Gegensatz zu den Formkörpern 32, 33 gemäß Figur 6 ist bei den Elektroden 36, 37 ein Kontaktmaterial 38 ganzflächig auf das dotierte Polymer aufgebracht. Dies hat den Vorteil, dass für die Kontaktierung eine größere Fläche zur Verfügung steht. Bei dem Kontaktmaterial 38 kann es sich beispielsweise um einen intrinsisch leitenden Kunststoff handeln.

### Bezugszeichenliste:

- 1: Formkörper
- 2: Formkörper
- 3: Elektrode
- 4: Elektrode
- 5: Küvette
- 6: Rahmen
- 7: Innenraum
- 8: Außenseite
- 9: Außenseite
- 10: Kontaktmaterial
- 11: Kontaktmaterial
- 12: Kontaktfläche
- 13: Kontaktfläche
- 30: Küvette
- 31: Grundkörper
- 32: Formkörper
- 33: Formkörper
- 34: Spalt
- 35: Kontaktmaterial
- 36: Elektrode
- 37: Elektrode
- 38: Kontaktmaterial

## Patentansprüche

1. Verfahren zur Herstellung zumindest eines elektrisch kontaktierbaren Bereichs auf einem mit einem leitfähigen Material dotierten Polymer, bei dem auf das Polymer ein Kontaktmaterial (10, 11, 35, 38) unter Druck aufgebracht wird, welches bei 23°C einen geringeren spezifischen Widerstand als das Polymer aufweist, **dadurch gekennzeichnet, dass** als Kontaktmaterial (10, 11, 35, 38) eine Folie unter Einwirkung von erhöhter Temperatur, die über der Erweichungstemperatur des dotierten Polymers liegt, auf das Polymer aufgebracht wird.

2. Verfahren nach Anspruch 1, **dadurch gekennzeichnet, dass** das Kontaktmaterial (10, 11, 35, 38) auf das Polymer aufgepresst wird.

3. Verfahren nach Anspruch 1 oder 2, **dadurch gekennzeichnet, dass** die Oberfläche des Polymers vor dem Aufbringen des Kontaktmaterials (10, 11, 35, 38) zumindest teilweise durch mechanische und/oder chemische Behandlung vergrößert wird.

4. Verfahren nach Anspruch 3, **dadurch gekennzeichnet, dass** die Oberfläche des Polymers durch mechanische Verletzung aufgeraut wird.

5. Verfahren nach einem der Ansprüche 1 bis 4, **dadurch gekennzeichnet, dass** das Kontaktmaterial (10, 11, 35, 38) einen spezifischen Widerstand bei 23°C unterhalb von 1 x 10⁻⁵ Ohm·cm, vorzugsweise von 1 x 10⁻⁶ bis 2 x 10⁻⁶ Ohm·cm, aufweist.

6. Verfahren nach einem der Ansprüche 1 bis 5, **dadurch gekennzeichnet, dass** das Kontaktmaterial (10, 11, 35, 38) eine Metallfolie, insbesondere eine Kupferfolie, oder eine Folie aus intrinsisch leitendem Kunststoff ist.

7. Verfahren nach einem der Ansprüche 1 bis 6, **dadurch gekennzeichnet, dass** das Kontaktmaterial (10, 11, 35, 38) mittels eines Prägestempels auf das Polymer aufgeprägt wird.

8. Verfahren nach Anspruch 7, **dadurch gekennzeichnet, dass** das Kontaktmaterial (10, 11, 35, 38) und zumindest ein Teil des Polymers auf eine Temperatur erwärmt werden, die oberhalb der Erweichungstemperatur des Polymers liegt, dass dann das Kontaktmaterial (10, 11, 35, 38) mittels des Prägestempels unter Druck auf die Oberfläche des Polymers gepresst wird, und dass der Prägestempel nach dem Abkühlen auf eine Temperatur, die unterhalb der Erweichungstemperatur des Polymers liegt, abgehoben wird.

9. Verfahren nach Anspruch 7 oder 8, **dadurch gekennzeichnet, dass** das Kontaktmaterial (10, 11, 35, 38) und/oder zumindest ein Teil des Polymers und/oder der Prägestempel auf eine Temperatur zwischen 100 und 300 °C erwärmt wird/werden.

10. Verfahren nach einem der Ansprüche 7 bis 9, **dadurch gekennzeichnet, dass** der Prägestempel mit einem Druck zwischen 50 und 100 N/mm² beaufschlagt wird.

11. Verfahren nach einem der Ansprüche 7 bis 9, **dadurch gekennzeichnet, dass** der Prägestempel mit einem Druck zwischen 100 und 500 N/mm² beaufschlagt wird..

12. Verfahren nach Anspruch 10 und 11, **dadurch gekennzeichnet, dass** der Prägestempel während der Prägephase zunächst mit einem Druck von 50 bis 100 N/mm², vorzugsweise 70 bis 90 N/mm², und dann, während der Abkühlphase, mit einem Druck von 100 bis 500 N/mm², vorzugsweise 350 bis 450 N/mm², beaufschlagt wird.

13. Verfahren nach einem der Ansprüche 7 bis 12, **dadurch gekennzeichnet, dass** das Aufprägen des Kontaktmaterials (10, 11, 35, 38) unter Wärmezufuhr zumindest teilweise im Vakuum oder in einer Stickstoffatmosphäre durchgeführt wird

14. Verfahren nach einem der Ansprüche 1 bis 13, **dadurch gekennzeichnet, dass** das Kontaktmaterial (10, 11, 35, 38) über eine haftvermittelnde Schicht, die vorzugsweise einen geringen spezifischen Widerstand aufweist, auf das Polymer aufgebracht wird.

15. Formkörper (1, 2, 32, 33) aus mit leitfähigem Material dotiertem Polymer, mit zumindest einem kontaktierbaren Bereich, innerhalb dessen auf das Polymer ein Kontaktmaterial (10, 11, 35, 38) aufgebracht ist, welches bei 23°C einen geringeren spezifischen Widerstand als das Polymer aufweist, **dadurch gekennzeichnet, dass** das Kontaktmaterial (10, 11, 35, 38) eine auf das Polymer heißgeprägte Folie ist.

16. Formkörper nach Anspruch 15, **dadurch gekennzeichnet, dass** das Kontaktmaterial (10, 11, 35, 38) ein Metall, vorzugsweise Kupfer, oder ein intrinsisch leitender Kunststoff ist und/oder dass das Kontaktmaterial einen spezifischen Widerstand bei 23°C unterhalb von 1 × 10⁻⁵ Ohm·cm, vorzugsweise von 1 × 10⁻⁶ bis 2 × 10⁻⁶ Ohm·cm, aufweist.

17. Formkörper nach Anspruch 15 oder 16, **dadurch gekennzeichnet, dass** zwischen dem Polymer und dem Kontaktmaterial (10, 11, 35, 38) eine kohlenstoffhaltige Schicht, vorzugsweise eine Graphitschicht, oder eine haftvermittelnde Schicht, die vorzugsweise einen geringen spezifischen Widerstand aufweist, angeordnet ist.

18. Formkörper nach einem der Ansprüche 15 bis 17, **dadurch gekennzeichnet, dass** das Polymer mit Kohlefasern, Graphit, Ruß und/oder Kohlenstoff-Nanotubes dotiert ist und dass die Dotierung in einer Konzentration von 50 bis 80 Gew.-% vorliegt.

19. Formkörper nach einem der Ansprüche 15 bis 18, **dadurch gekennzeichnet, dass** das Polymer Polycarbonat, Polyetheretherketon, Polypropylen, Polyamid vorzugsweise Polyamid 6 oder Polyamid 66, Polyphenylensulfid oder ein Gemisch dieser Polymere ist oder eines oder mehrere dieser Polymere als Hauptbestandteil enthält.

20. Formkörper nach einem der Ansprüche 16 bis 19, **dadurch gekennzeichnet, dass** der intrinsisch leitende Kunststoff ein Polyanilin, Polyacetylen, Poly-para-phenylen, Poly-para-phenylensulfid, Polypyrrol oder Polythiophen ist oder eines oder mehrere dieser Polymere als Hauptbestandteil enthält

21. Formkörper nach einem der Ansprüche 15 bis 20, **dadurch gekennzeichnet, dass** dieser als Elektrode (3, 4, 36, 37) ausgebildet ist.

22. Formkörper nach einem der Ansprüche 15 bis 21, **dadurch gekennzeichnet, dass** dieser, vorzugsweise in Form einer Elektrode, Bestandteil einer Küvette oder zumindest eines Reaktionsraums einer Multiwell-Platte ist.

## Claims

1. Method for generating at least one electrically contactable area on a polymer which is doped with a conductive substance, wherein a contact material (10, 11, 35, 38) is applied under pressure onto said polymer, said contact material having a lower specific resistance at 23°C than said polymer, **characterized in that** the contact material (10, 11, 35, 38) is a foil which is applied onto said polymer while being exposed to a temperature, which is higher than the softening temperature of the doped polymer.

2. Method according to claim 1, **characterized in that** said contact material (10, 11, 35, 38) is applied onto said polymer exerting pressure.

3. Method according to claim 1 or 2, **characterized in that** the surface of said polymer is at least partially enlarged by mechanical and/or chemical treatment before said contact material (10, 11, 35, 38) is applied.

4. Method according to claim 3, **characterized in that** said surface of said polymer is roughened by mechanical invasion.

5. Method according to any one of claims 1 to 4, **characterized in that** said contact material (10, 11, 35, 38) has a specific resistance at 23°C below 1 x 10⁻⁵ Ohm·cm, preferably between 1 x 10⁻⁶ and 2 x 10⁻⁶ Ohm·cm.

6. Method according to any one of claims 1 to 5, **characterized in that** said contact material (10, 11, 35, 38) is a metal foil, in particular a copper foil, or a foil made of an intrinsically conductive plastic material.

7. Method according to any one of claims 1 to 6, **characterized in that** said contact material (10, 11, 35, 38) is embossed onto said polymer using an embossing die.

8. Method according to claim 7, **characterized in that** said contact material (10, 11, 35, 38) and at least a part of said polymer are heated to a temperature which is higher than the softening temperature of said polymer, that said contact material (10, 11, 35, 38) is subsequently applied onto the surface of said polymer under pressure using an embossing die, and that said embossing die is lifted after cooling down to a temperature which is lower than the softening temperature of said polymer.

9. Method according to claim 7 or 8, **characterized in that** said contact material (10, 11, 35, 38) and/or at least one part of said polymer and/or said embossing die is/are heated to a temperature between 100 and 300°C.

10. Method according to any one of claims 7 to 9, **characterized in that** a pressure between 50 and 100 N/mm² is applied to said embossing die.

11. Method according to any one of claims 7 to 9, **characterized in that** a pressure between 100 and 500 N/mm² is applied to said embossing die.

12. Method according to claim 10 or 11 **characterized in that** at first, while embossing, a pressure between 50 and 100 N/mm², preferably 70 and 90 N/mm², and subsequently, during the cooling period, a pressure between 100 and 500 N/mm², preferably 350 and 450 N/mm², is applied to said embossing die.

13. Method according to any one of claims 7 to 12, **characterized in that** embossing of said contact material (10, 11, 35, 38) takes place under heat and, at least temporarily, in a vacuum or in a nitrogen atmosphere.

14. Method according to any one of claims 1 to 13, **characterized in that** said contact material (10, 11, 35, 38) is applied onto said polymer by an adhesion-mediating layer which preferably has a low specific resistance.

15. Formed body (1, 2, 32, 33) made of a polymer which is doped with a conductive substance, which has at least one contactable area, within which a contact material (10, 11, 35, 38) is applied onto said polymer, wherein said contact material (10, 11, 35, 38) has a lower specific resistance at 23°C than said polymer, **characterized in that** said contact material (10, 11, 35, 38) is a foil, which is hot embossed onto the polymer.

16. Formed body according to claim 15, **characterized in that** said contact material (10, 11, 35, 38) is a metal, preferably copper, or an intrinsically conductive plastic material, and/or that said contact material (10, 11, 35, 38) has a specific resistance at 23°C below 1 x 10⁻⁵ Ohm·cm, preferably between 1 x 10⁻⁶ and 2 x 10⁻⁶ Ohm·cm.

17. Formed body according to claim 15 or 16, **characterized in that** a carbon-based material, preferably a graphite sheet, or an adhesion-mediating layer having a low specific resistance, is disposed between said polymer and said contact material (10, 11, 35, 38).

18. Formed body according to any one of claims 15 to 17, **characterized in that** said polymer is doped with carbon fibres, graphite, carbon black and/or carbon nanotubes, and that the overall concentration of said dope in said polymer is between 50 and 80 % w/w.

19. Formed body according to any one of claims 15 to 18, **characterized in that** said polymer is polycarbonate, polyetheretherketone, polypropylene, polyamide, preferably polyamide 6 or polyamide 66, polyphenylensulfide or a mixture of these polymers, or at least based on one or several of these polymers.

20. Formed body according to any one of claims 16 to 19, **characterized in that** said intrinsically conductive plastic material is polyaniline, polyacetylene, poly-paraphenylene, poly-para-phenylensulfide, polypyrroles or poly-thiophene , or at least based on one or several of these polymers.

21. Formed body according to any one of claims 15 to 20, **characterized in that** said formed body (1, 2, 32, 33) acts as an electrode (3, 4, , 36, 37) .

22. Formed body according to any one of claims 15 to 21, **characterized in that** said formed body (1, 2, 32, 33) is part of a cuvette or at least of one reaction chamber of a multiwell plate, preferably in the form of an electrode.

## Revendications

1. Procédé de fabrication d'au moins une zone susceptible d'être mise en contact électrique sur un polymère dopé avec une matière conductrice, du type selon lequel sur le polymère est appliqué sous pression un matériau de contact (10, 11, 35, 38), qui présente à 23°C une résistivité électrique plus limitée que celle du polymère, **caractérisé en ce qu'**en tant que matériau de contact (10, 11, 35, 38) est appliqué sur le polymère un film sous l'action d'une température élevée, qui se situe au-dessus de la température de ramollissement du polymère.

2. Procédé selon la revendication 1, **caractérisé en ce que** le matériau de contact (10, 11, 35, 38) est pressé sur le polymère.

3. Procédé selon la revendication 1 ou 2, **caractérisé en ce que** la surface du polymère est, avant l'application du matériau de contact (10, 11, 35, 38), agrandie au moins partiellement par traitement mécanique et/ou chimique.

4. Procédé selon la revendication 3, **caractérisé en ce que** l'on confère de la rugosité à la surface du polymère par traitement mécanique de scarification, de lacération ou analogue.

5. Procédé selon l'une des revendications 1 à 4, **caractérisé en ce que** le matériau de contact (10, 11, 35, 38) présente une résistivité électrique à 23°C inférieure à 1 x 10⁻⁵ Ohm.cm, de préférence allant de 1 x 10⁻⁶ Ohm. cm jusqu'à 2 x 10⁻⁶ Ohm. cm.

6. Procédé selon l'une des revendications 1 à 5, **caractérisé en ce que** le matériau de contact (10, 11, 35, 38) est un film métallique, en particulier un film de cuivre, ou un plastique à conductibilité intrinsèque.

7. Procédé selon l'une des revendications 1 à 6, **caractérisé en ce que** le matériau de contact (10, 11, 35, 38) est estampé sur le polymère au moyen d'un poinçon d'estampage.

8. Procédé selon la revendication 7, **caractérisé en ce que** le matériau de contact (10, 11, 35, 38) et au moins une partie du polymère sont chauffés à une température qui se situe au-dessus de la température de ramollissement du polymère, de telle façon qu'ensuite le matériau de contact (10, 11, 35, 38) soit pressé au moyen du poinçon d'estampage, par pression sur la surface du polymère, et que le poinçon d'estampage soit éloigné par relèvement, après refroidissement jusqu'à une température qui se situe au-dessous de la température de ramollissement du polymère.

9. Procédé selon la revendication 7 ou 8, **caractérisé en ce que** le matériau de contact (10, 11, 35, 38) et/ou au moins une partie du polymère et/ou le poinçon d'estampage est/sont chauffé(s) à une température comprise entre 100 et 300°C.

10. Procédé selon l'une des revendications 7 à 9, **caractérisé en ce que** le poinçon d'estampage est actionné avec une pression comprise entre 50 et 100 N/mm².

11. Procédé selon l'une des revendications 7 à 9, **caractérisé en ce que** le poinçon d'estampage est actionné avec une pression comprise entre 100 et 500 N/mm².

12. Procédé selon la revendication 10 et 11, **caractérisé en ce que** le poinçon d'estampage pendant la phase initiale d'estampage est actionné avec une pression allant de 50 à 100 N/mm², de préférence de 70 à 90 N/mm² et **en ce qu'**ensuite pendant la phase de refroidissement il est actionné avec une pression allant de 100 à 500 N/mm², de préférence allant de 350 à 450 N/min².

13. Procédé selon l'une des revendications 7 à 12, **caractérisé en ce que** l'estampage du matériau de contact (10, 11, 35, 38) est réalisé avec apport de chaleur, au moins partiellement sous vide ou dans une atmosphère d'azote.

14. Procédé selon l'une des revendications 1 à 13, **caractérisé en ce que** le matériau de contact (10, 11, 35, 38) est implanté sur le polymère par le biais d'une couche de couplage par collage, qui présente de préférence une résistivité électrique limitée.

15. Corps de formage (1, 2, 32, 33) à base de polymère dopé avec une matière conductrice, avec au moins une zone de contact possible, à l'intérieur de laquelle est appliqué sur le polymère un matériau de contact (10, 11, 35, 38), qui présente à 23°C une résistivité électrique plus limitée que celle du polymère, **caractérisé en ce que** le matériau de contact (10, 11, 35, 38) est un film estampé à chaud sur le polymère.

16. Corps de formage selon la revendication 15, **caractérisé en ce que** le matériau de contact (10, 11, 35, 38) est un métal, de préférence le cuivre, ou un plastique à conductibilité intrinsèque et/ou en ce que le matériau de contact présente une résistivité électrique à 23°C inférieure à 1 x 10⁻⁵Ohm.cm, de préférence allant de 1 x 10⁻⁶ Ohm. cm à 2 x 10⁻⁶ Ohm. cm.

17. Corps de formage selon la revendication 15 ou 16, **caractérisé en ce qu'**entre le polymère et le matériau de contact (10, 11, 35, 38) est disposée une couche contenant du carbone, de préférence une couche en graphite, ou une couche de couplage par collage, qui présente de préférence une résistivité électrique limitée.

18. Corps de formage selon l'une des revendications 15 à 17, **caractérisé en ce que** le polymère est dopé avec des fibres de carbone, du graphite, du noir de fumée et/ou des nanotubes de carbone et **en ce que** la dotation en matière conductrice dopante est présente en une concentration allant de 50 à 80 % en poids.

19. Corps de formage selon l'une des revendications 15 à 18, **caractérisé en ce que** le polymère est du polycarbonate, du polyétheréthercétone, du polypropylène, du polyamide, de préférence du polyamide 6 ou du polyamide 66, du polyphénylènesulfone ou un mélange de ces polymères ou **en ce qu'**il contient comme constituant principal un ou plusieurs de ces polymères.

20. Corps de formage selon l'une des revendications 16 à 19, **caractérisé en ce que** le plastique à conductibilité intrinsèque est de la polyaniline, du polyacétylène, du poly-paraphénylène, du polyparaphénylènesulfone, du polypyrrol ou du polythiophène ou **en ce qu'**il contient comme constituant principal un ou plusieurs de ces polymères.

21. Corps de formage selon l'une des revendications 15 à 20, **caractérisé en ce que** celui-ci est conformé en tant qu'électrode (3, 4, 36, 37).

22. Corps de formage selon l'une des revendications 15 à 21, **caractérisé en ce que** celui-ci, de préférence sous forme d'une électrode, est un constituant d'une cuvette ou d'au moins un espace réactionnel d'une plaque multi-puits.
